# EUROPEAN PATENT SPECIFICATION

(11) **EP 1 543 104 B1**
(45) Date of publication and mention of the grant of the patent: **18.04.2007**
(21) Application number: 03748039.9
(22) Date of filing: 16.09.2003
(51) Int. Cl.: C12N 1/21, C12P 17/12

(54) **MICROORGANISM AND PROCESS FOR PREPARING VITAMIN B6**
MIKROORGANISMUS UND VERFAHREN FÜR DIE PRODUKTION VON VITAMIN B6
MICRO-ORGANISME ET PROCEDE DE PREPARATION DE LA VITAMINE B6

(30) Priority: 27.09.2002 EP 02021621
(43) Date of publication of application: 22.06.2005
(73) Proprietor: DSM IP Assets B.V., 6411 TE Heerlen (NL)
(72) Inventor: HOSHINO, Tatsuo, Kanagawa-ken, Kanagawa 248-0027 (JP); ICHIKAWA, Keiko, Marin-terasu-nibankan 201, Kanagawa-ken, Kanagawa 251-0047 (JP); NAGAHASHI, Yoshie, Grand Hill Tenjin 207, Kanagawa-ken, Kanagawa 252-0814 (JP); TAZOE, Masaaki, Yokohama-shi, Kanagawa 235-0045 (JP)
(74) Representative: Keller, Günter
(86) International application number: PCT/EP2003/010286
(87) International publication number: WO 2004/029228

(56) References cited:
- EP-A- 0 765 938
- TAZOE M ET AL: "Biosynthesis of vitamin B6 in Rhizobium" JOURNAL OF BIOLOGICAL CHEMISTRY, AMERICAN SOCIETY OF BIOLOGICAL CHEMISTS, BALTIMORE, MD, US, vol. 275, no. 15, 14 April 2000 (2000-04-14), pages 11300-11305, XP002221306 ISSN: 0021-9258
- YOUNG J M: "The genus name Ensifer Casida 1982 takes priority over Sinorhizobium Chen et al. 1988, and Sinorhizobium morelense Wang et al. 2002 is a later synonym of Ensifer adhaerens Casida 1982. Is the combination 'Sinorhizobium adhaerens' (Casida 1982) Willems et al. 2003 legitimate? Request for an Opinion" INTERNATIONAL JOURNAL OF SYSTEMATIC AND EVOLUTIONARY MICROBIOLOGY 2003 UNITED KINGDOM, vol. 53, no. 6, 30 May 2003 (2003-05-30), pages 2107-2110, XP002268768 ISSN: 1466-5026

### SEQUENCE LISTING

<110> Roche Vitamins AG
<120> Microorganism and process for preparing vitamin B6
<130> NDR5216
<160> 4
<170> PatentIn version 3.1
<210> 1
   <211> 24
   <212> DNA
   <213> Artificial
<400> 1
   tcccatatgc ctgcaaagct ctcc
<210> 2
   <211> 24
   <212> DNA
   <213> Artificial
<400> 2
   tccctgcagt taagccgtct cgcc
<210> 3
   <211> 20
   <212> DNA
   <213> Artificial
<400> 3
   cctgcaggca ggagatctat
<210> 4
   <211> 20
   <212> DNA
   <213> Artificial
<400> 4
   cctgcagacg ctgcttgcgt

## Description

The present invention relates to novel recombinant microorganisms and a process for preparing vitamin B₆ using the same.

"Vitamin B₆" as used in this invention includes pyridoxol, pyridoxal, and pyridoxamine. Vitamin B₆ is a vitamin indispensable to human beings or other animals and used as a raw material of medicines or as feed additives.

The biosynthetic pathway of vitamin B₆ in *Escherichia coli* and *Sinorhizobium meliloti* has been well elucidated. Pyridoxol 5'-phosphate (referred to as PNP hereinafter) is thought to be synthesized from two precursors, 1-deoxy-D-xylulose 5-phosphate (referred to as DXP hereinafter) and 4-(phosphohydroxy)-L-threonine (referred to as HTP hereinafter) by two enzymes, HTP dehydrogenase and PNP synthase encoded by pdxJ gene.

In *E*. *coli,* HTP is thought to be synthesized from D-erythrose 4-phosphate (E4P) by a three step reaction. The first step reaction, oxidation of E4P to D-erythronate 4-phosphate (referred to as ENP hereinafter), is catalyzed by E4P dehydrogenase encoded by epd.

But, according to search of the genome database of *S*. *meliloti* strain 1021, no homologue of epd of *E. coli* is detected. Furthermore, there has been no report about E4P dehydrogenase in *S*. *meliloti* so far. It is, therefore, considered that *S*. *meliloti* has different biosynthetic pathway of HTP from that of *E*. *coli.* On the other hand, *S*. *meliloti* IFO 14782 accumulates a large amount of protocatechuate (one of shikimic acid derivatives), which might be synthesized from E4P.

The above findings suggest that stimulation of the conversion of E4P to ENP by incorporation of epd in *S*. *meliloti* leads to a development of an additional biosynthetic pathway of vitamin B₆ and an increase of vitamin B₆ production in *S*. *meliloti.* But in fact, vitamin B₆ production of *S*. *meliloti* was not stimulated by incorporation of only epd gene.

On the other hand, when both epd and pdxJ genes were incorporated in *S*. *meliloti,* vitamin B₆ production was considerably improved.

According to the present invention, it is possible to improve the production efficiency of vitamin B₆ drastically by fermentation using a microorganism of the genus *Sinorhizobium* having a recombinant plasmid comprising a vector containing epd and pdxJ. Vitamin B₆ can advantageously be produced in the culture broth by cultivating said microorganism, and can be recovered therefrom in a desired purity.

The present invention provides a recombinant microorganism, e.g., a member of the genus *Sinorhizobium* capable of producing vitamin B₆ comprising a plasmid with pdxJ and epd.

The present invention also provides a process for producing vitamin B₆ which comprises cultivating said microorganism in a culture medium so that vitamin B₆ is produced and accumulated in the culture broth and collecting the produced vitamin B₆.

A pdxJ of *E*. *coli* is reported to be the gene encoding PNP synthase catalyzing synthesis of PNP from DXP and aminoacetone 3-phosphate. As used herein, reference to a "pdxJ" means the natural gene itself as well as any functional equivalent thereof. A functional equivalent of pdxJ is, therefore, any gene, which encodes an enzyme, PNP synthase. A functional equivalent of pdxJ can be isolated from any organism, such as, but not limited to, *Klebsiella pneumoniae* and *Pseudomonas aeruginosa* other bacteria, yeast, and plant.
The pdxJ used in the present invention is preferably derived from microorganisms of the genus *Sinorhizobium* but any gene functional equivalent thereof can be used in the present invention. For example, a DNA of pdxJ derived from *S*. *meliloti* IFO 14782 can be cloned in the following manner.

The primers for polymerase chain reaction (referred to as PCR hereinafter) are synthesized in accordance with the DNA sequence of pdxJ in a DNA database of *S*. *meliloti* strain 1021, and which contain restriction enzyme recognition site at the 5' end of each primer. The gene, pdxJ, can be amplified by PCR using the primers and chromosomal DNA of *S*. *meliloti* IFO 14782. Amplified pdxJ is ligated into a vector replicable in *E*. *coli* such as available pUC series or pBR series. A plasmid, wherein pdxJ is inserted, can be selected by agarose gel analysis of the plasmid digested with endonuclease, and the sequence of amplified region can be ascertained with a DNA sequencer.

An epd referred to herein means the gene encoding E4P dehydrogenase catalyzing oxidation of E4P to ENP and a functional equivalent thereof of the *E*. *coli* epd is, therefore, any gene, which encodes an active E4P dehydrogenase. A functional equivalent of the *E*. *coli* epd can be isolated from any organism, such as ,but not limited to, *Vibrio cholerae, Pseudomonas aeroginosa,* other bacteria, yeast, and plant. For example, epd, which derived from *E. coli* K12, can be cloned by using PCR in a similar way as mentioned above.

As vector for incorporation of recombinant DNA in *S*. *meliloti,* two types of vectors can be used. One is a replicable, broad-host range vector, such as pVK100, pRK290, pLAFR1 or RSF1010. The other is an integration vector, such as pSUP202.

A vector for expressing recombinant protein in *S*. *meliloti* can be provided by inserting a DNA fragment encoding a promoter functioning in *S*. *meliloti,* such as ptac, plac, ptrc, pS1 (promoter of small ribosomal subunit of *S*. *meliloti),* or pNm (promoter of neomycin resistant gene) and either pdxj or epd or both of them into a vector.

The procedure for constructing such recombinant vectors can be performed according to standard techniques known in the fields of molecular biology, bioengineering, and genetic engineering.

For example, pdxJ may be placed in pVK100 under the control of ptrc promoter to construct pVK601. In the other embodiment, epd is placed in pVK100 under the control of ptac promoter to construct pVK602 and both pdxJ and epd are placed in pVK100 under the control of ptrc promoter and ptac promoter, respectively, to construct pVK611.

As a parent strain for preparing the recombinant microorganisms constructed in the present invention, any strains belonging to the genus *Sinorhizobium* can be used, and the microorganisms belonging to the genus *Sinorhizobium* may be isolated from natural sources, or may be purchased from culture collections, such as Institute for fermentation, Osaka (IFO), Japan. Preferably, *S*. *meliloti* IFO 14782, which is deposited at the Deutsche Sammlung von Mikroorganismen und Zellkulturen GmbH (DSM) in Göttingen, Germany under DSM 10226, deposited on September 4, 1995, can be used for the present invention.

Microorganisms showing drastically increased productivity of vitamin B₆ can be constructed by introducing a recombinant vector being incorporated with pdxJ and epd. For example, such a recombinant microorganism indicating drastically increased productivity of vitamin B₆, which are derived from *S*. *meliloti* IFO 14782 is constructed as described below. A recombinant vector constructed by incorporating either pdxJ, epd, or both of them can be introduced into *S*. *meliloti* IFO14782 by tri-parental mating in the following manner. *S*. *meliloti* as a recipient strain, *E*. *coli* harboring a helper plasmid as a helper strain, and *E*. *coli* harboring a recombinant vector as a donor strain are cultivated separately and mixed together. After mix cultivation on plate, *S*. *meliloti* receiving the recombinant vector from a donor strain can be selected on agar plate containing appropriate antibiotics.

The recombinant strain carrying the plasmid is selected by the preparation of the plasmid from the colonies grown on the plates and examination by endonuclease digestion. The recombinant strain, which recombinant DNA is integrated in chromosome, is selected by the preparation of chromosome DNA from the colonies grown on the plates and detection the integrated DNA by Southern hybridization.

The expression of the pdxJ and epd incorporated into the plasmid in *S*. *meliloti* can be analyzed by culturing the resultant recombinant strain in a medium, and preparing cell-free extract and subjected SDS-polyacrylamide gel electrophoresis (SDS-PAGE).

The recombinant microorganisms obtained in the present invention are incubated in a medium containing an assimilable carbon source, a digestible nitrogen source, an inorganic salt, and other nutrients necessary for their growth. As a carbon source, e.g., glucose, fructose, lactose, maltose, galactose, sucrose, starch, dextrin, or glycerol may be employed. As a nitrogen source, e.g., peptone, corn steep liquor, soybean powder, yeast extract, meat extract, ammonium chloride, ammonium sulfate, ammonium nitrate, urea, or their mixture thereof may be employed.

Further, for trace elements, sulfates, hydrochlorides, or phosphates of calcium, magnesium, zinc, manganese, cobalt, and iron may be employed. And, if necessary, conventional nutrient factors, a trapping agent of phosphate ion, or an antifoaming agent, such as magnesium carbonate, aluminum oxide, allophane, animal oil, vegetable oil, or mineral oil can also be added supplementary in a fermentation medium.

The pH of the culture medium may be about 5.0 to 9.0, preferably 6.5 to 7.5. The cultivation temperature may be about 10 °C to 40 °C, preferably 25 °C to 35 °C. The cultivation time may be about 1 day to 15 days, preferably 2 days to 9 days. In the cultivation, aeration and agitation usually give favorable results.

After the cultivation, vitamin B₆ produced may be separated from the culture broth and purified. For this purpose a process generally used for extracting a certain product from the culture broth may be applied by utilizing various properties of vitamin B₆. Thus, for example, the cells are removed from the culture broth, the desired substance in the filtrate is absorbed on active carbon, then eluted and purified further with an ion exchange resin. Alternatively, the culture filtrate is applied directly to an ion exchange resin and, after the elution, the desired product is recrystallized from mixture of alcohol and water. The amount of vitamin B₆ produced in culture broth can be quantified by high pressure liquid chromatography (HPLC).

The present invention will be explained more in detail by referring to the following Examples.

*E. coli* K12 was used as a source of chromosome DNA for the epd gene cloning experiment. *S. meliloti* IFO14782 was used as a source of chromosome DNA for pdxJ gene cloning experiment and as a host strain for evaluating vitamin B₆ fermentation by recombinants. Strains of *E*. *coli* were cultured in a medium (referred to as LB hereinafter) consisting of 1 % Bacto Tryptone (Becton Dickinson Microbiology systems, MD, USA), 0.5 % Bacto Yeast extract (Becton Dickinson Microbiology systems, MD, USA) and 0.5 % NaCl.
Bacto-agar (1.5%) was added to the LB medium for preparing agar plates.
Plasmid DNA was isolated from *E*. *coli* or *S. meliloti* with QIAGEN Midi kit (QIAGEN GmbH, Germany) or with Automatic DNA Isolation System PI-50 (Kurabo Industry Ltd., Japan). Chromosomal DNA was isolated using QIAGEN genomic-tips.
Restriction enzymes, alkaline phosphatase, ligation kit, *E*. *coli* JM109, HB101 competent cells (Takara Bio. Inc, Shiga, Japan), TOPO TA cloning kit (Invitrogen Japan K.K., Japan) were used according to the producer's instructions.
Plasmid pKK223-3, pTrc99A, and SureClone ligation kit were purchased from Amersham Biosciences Corp. (NJ, USA). For restriction enzyme analysis, the DNA fragments were fractionated in agarose gels (1.0 %) and isolated from the gels by means of extraction using a commercially available system with QIAEXII (QIAGEN GmbH, Germany). DNA sequence was determined with an ALF DNA sequencer (Amersham Biosciences Corp., NJ, USA).

### Example 1: Cloning of pdxJ of S. meliloti and epd of E. coli

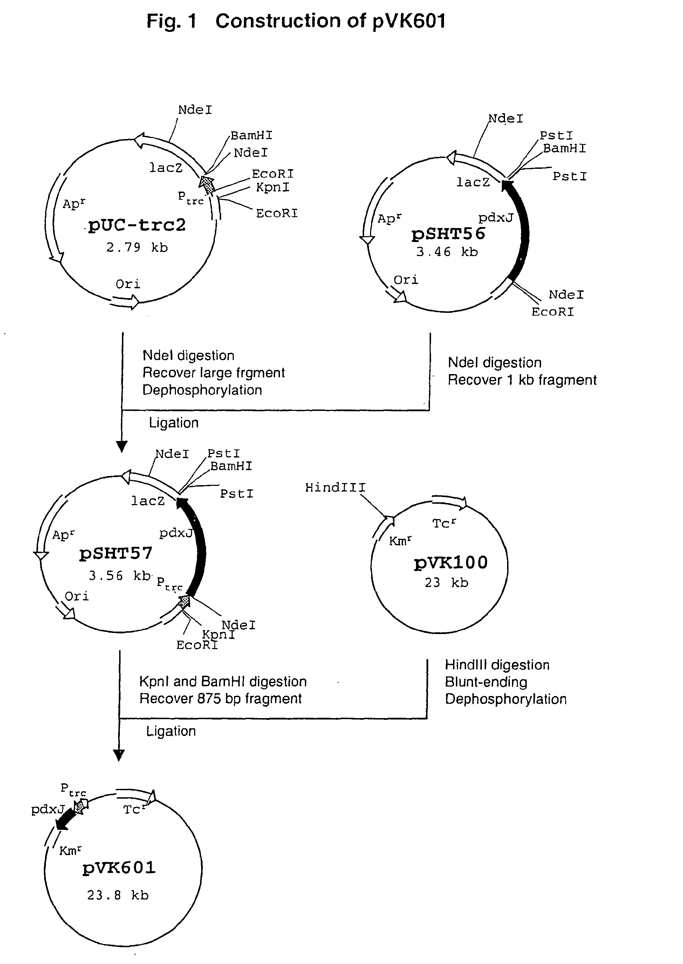

(a) pdxJ from *S. meliloti* IFO 14782 chromosome
   To amplify pdxJ of *S. meliloti* IFO 14782 using PCR method, two primers were synthesized according to the DNA sequence of pdxJ (2249854-2250606, complement) in the genome database of *S. meliloti* strain 1021 (Accession No. NC_003047): primer A (SEQ ID NO:1) and primer B (SEQ ID NO:2).
   Chromosomal DNA was extracted from the cells grown in a medium (referred to as LBMC hereinafter) composed of 1 % Bacto Tryptone (Becton Dickinson Microbiology systems, MD, USA), 0.5 % Bacto Yeast extract (Becton Dickinson Microbiology systems, MD, USA), 0.5 % NaCl, 0.061 % MgSO₄·7H₂O, and 0.036 % CaCl₂·2H₂O with QIAGEN genomic-tips. PCR was performed using advantage-HF PCR kit (Clontech Laboratories Inc. CA USA).
   100 µl of reaction mixture contained 10 ng of chromosomal DNA of *S*. *meliloti* IFO 14782, 50 pmol of the two primers, 10 µl of 10 x HF dNTP mix, 10 µl of appended 10 x HF PCR reaction buffer, and 2 µl of 50 x advantage-HF polymerase mix. The reaction condition was as follows; holding at 94°C for 3 min, 4 cycles of 30 sec at 98°C, 1 min at 53°C, 1 min at 72°C, 20 cycles of 30 sec at 98°C, 1 min 68°C, and holding at 72°C for 10 min. 10 µl of reaction mixture was subjected to agarose gel on 1 % (w/v) and a DNA band of 770 bp was recovered from the gel with QIAEXII.
   The fragment was ligated to pUC18, which was digested with Smal and dephosphorylated with alkaline phosphatase, by using SureClone ligation kit. Thus obtained ligation mixture was transformed into *E. coli* JM109 competent cells and plated on plates of LB medium containing 100 µg/ml of ampicillin (referred to as Amp hereinafter). Plasmids of colonies grown on the plates were prepared with a DNA automatic isolation system.
   By analysis of the plasmid with restriction enzyme, a recombinant plasmid pSHT56, wherein pdxJ was the same direction as lacZ gene on pUC18, was obtained. pSHT56 was prepared from *E*. *coli* JM109 harboring pSHT56 with QIAGEN plasmid Midi kit. The DNA sequence of pdxJ in the plasmid was ascertained with an ALF DNA sequencer and it confirmed to be identical with that of a genome database of *S*. *meliloti* strain 1021.
   The function of pdxJ gene cloned from *S*. *meliloti* IFO 14782 was confirmed by the following method. As a vector for expression of pdxJ in *E*. *coli,* pUC 18 was remodeled into pUC-trc2, which has trc promoter region of pTrc99A followed by Ndel recognition sequence. pUC-trc2 was prepared from *E*. *coli* JM 109 harboring pUC-trc2 with QIAGEN plasmid Midi kit. To give an expression plasmid for pdxJ in *E*. *coli,* pUC-trc2 was digested with Ndel, and a 2.5-kb fragment was recovered from agarose gel and dephosphorylated with alkaline phosphatase.
   pSHT56 was cleaved with NdeI, subjected to agarose gel and resulting 1-kb fragment was recovered from the gel with QIAEXII. The recovered 1-kb fragment was ligated to prescribed 2.5-kb fragment of pUC-trc2 with ligation kit. *E*. *coli* JM109 was transformed with thus-obtained ligation mixture and plated on LB plates containing 100 µg/ml of Amp. Plasmid of a colony grown on the plate was prepared with an automatic DNA isolation system.
   By analysis of the plasmid with restriction enzymes, a recombinant plasmid pSHT57, wherein pdxJ was incorporated into the same direction as trc promoter, was obtained (Fig. 1). pSHT57 was prepared from *E*. *coli* JM109 harboring pSHT57 with QIAGEN plasmid midi kit.
(b) epd from *E*. *coli* K-12 chromosome
   The epd gene was amplified from 100 ng of chromosomal DNA of *E*. *coli* K-12 with advantage-HF PCR kit using 10-pmol of two primers, i.e. Primer C (SEQ ID NO:3) and Primer D (SEQ ID NO:4).
   Reaction condition was as follows; after holding 15 sec at 94°C, 25 cycles of 15 sec at 94°C, 3 min at 68°C. The amplified 1.0-kb fragment was directly cloned in pCRII-TOPO vector with TOPO TA cloning kit. Sequence of amplified region was ascertained to be identical with the CDS region of epd (3070692-3071711, complement) in accession number NC_000913 and obtained plasmid was named pCRepd.

### Example 2: Construction of recombinant plasmids

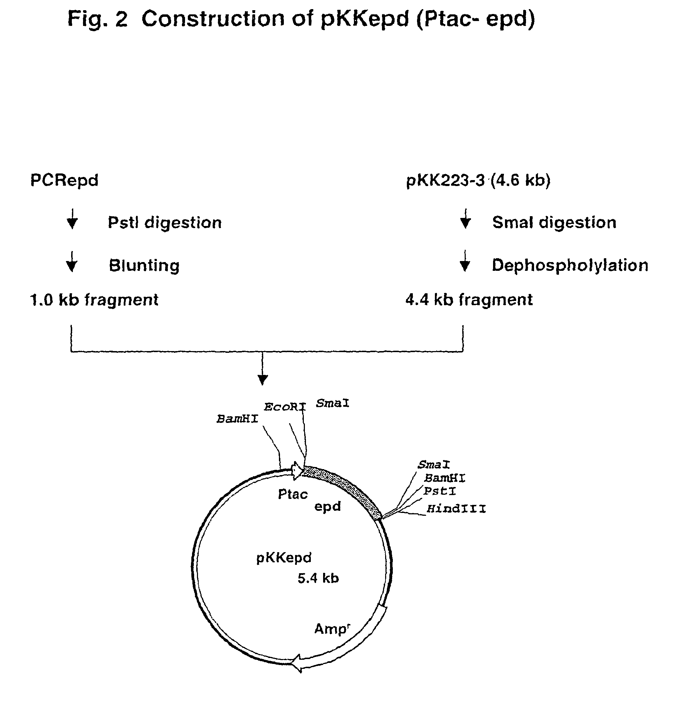

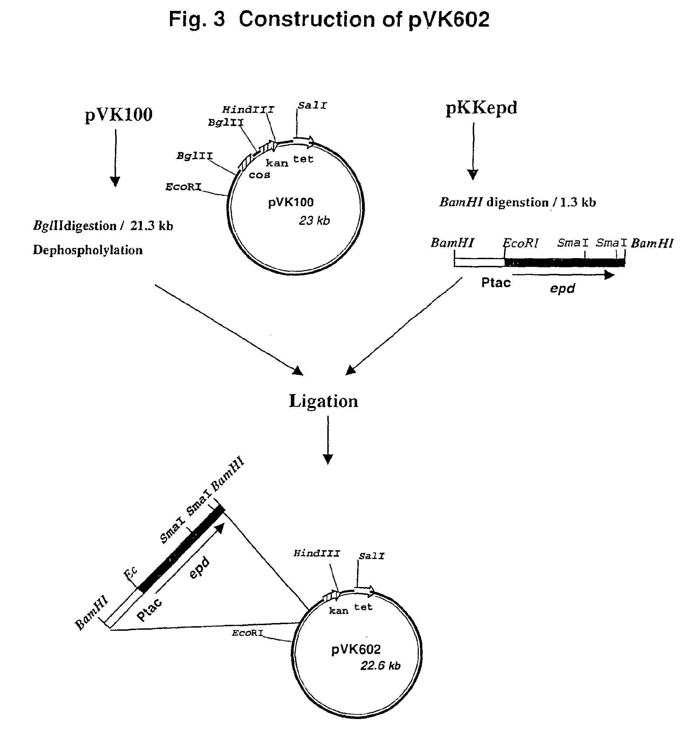

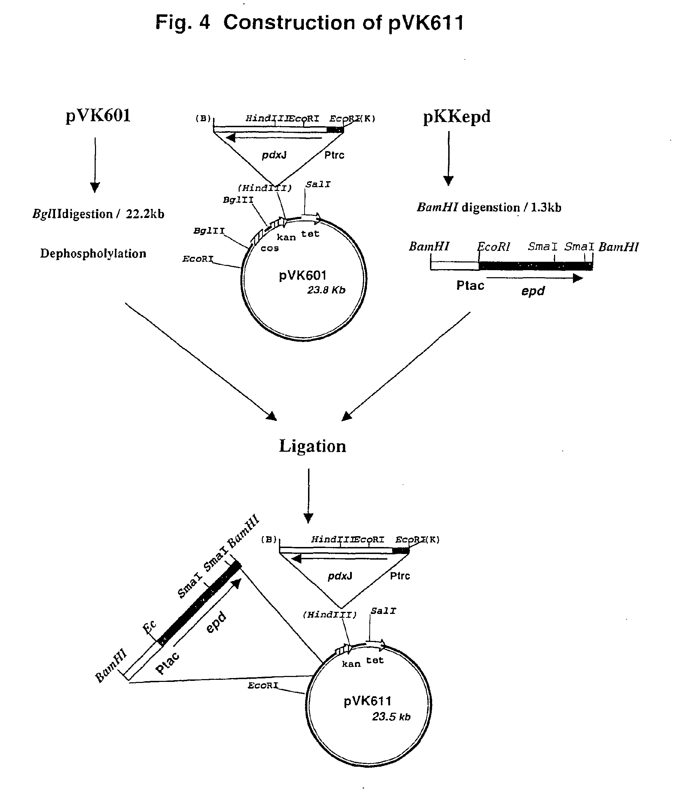

(a) pVK 601
   To construct an expression vector in *S*. *meliloti* IFO 14782, pVK100 was used, which is reported to be a broad host range vector, IncP-1 type, and replicable in *S*. *meliloti.* pVK100 was prepared from *E*. *coli* HB101/pVK100 with QIAGEN plasmid midi kit, and digested with HindIII, blunt-ended by blunting kit and dephosphorylated with alkaline phosphatase. pSHT57 was digested with BamHI and KpnI.
   Resulting 875-bp fragment, which contained trc promoter and pdxJ, was recovered from agarose gel, blunt-ended, and ligated to prescribed pVK100 with ligation kit. *E*. *coli* HB101 competent cells were transformed with the obtained ligation mixture and plated on LB plates containing 10 µg/ml of tetracycline (referred to as Tc hereinafter). Plasmids of colonies grown on the plates were prepared with an automatic DNA isolation system.
   By analysis of the plasmid with restriction enzyme, a recombinant plasmid, pVK601, wherein trc promoter and pdxJ were the opposite direction against kanamycin (referred to as Km hereinafter) resistant gene, was obtained (Fig. 1).
(b) pVK 602
   To amplify epd in *S*. *meliloti,* tac promoter driven epd cassette was constructed. 1.0-kb PstI fragment from pCRepd [Example 1-(b)] was blunted and ligated into SmaI site of pKK223-3 in an orientation that allowed transcription of epd from tac promoter and resulting plasmid was named pKKepd (Fig. 2). Then mobilizable cosmid pVK100 was digested with BglII, then about 21.3-kb fragment were recovered.
   After the fragment was treated with bacterial alkaline phosphatase, 1.3-kb BamHI fragments from pKKepd were ligated into the BglII digested and dephosphorylated fragment to give a plasmid pVK602 (Fig. 3).
(c) pVK611
   In order to co-express both epd of *E*. *coli* and pdxJ of *S*. *meliloti* in *S. meliloti,* pVK611 was constructed in a similar manner in described in Example 2-(b). pVK601 was digested with BglII and about 22.2-kb fragments were recovered. After the fragments were treated with bacterial alkaline phosphatase, 1.3-kb BamHI fragments from pKKepd was ligated into the BglII digested and dephosphorylated fragment to give plasmid pVK611 (Fig. 4).

### Example 3: Introduction of recombinant plasmids into S. meliloti IFO14782

The plasmids were transferred from *E. coli* HB101 to *S*. *meliloti* IFO14782 with the help of *E. coli* HB101 carrying pRK2013 (Km^{r}, IncP, tra⁺, CoIEI ori (ATCC37159) by using tri-parental conjugal mating method as described below. *S. meliloti* IFO14782 as a recipient strain was inoculated in liquid LBMC medium and incubated with shaking at 30°C at 140 rpm for 16 hours.
*E. coli* HB101 harboring pRK2013 as a helper strain and *E*. *coli* HB101 harboring pVK601 as a donor strain were inoculated in liquid LB medium containing 50 µg/ml of Km and liquid LB medium containing 10 µg/ml of Tc, respectively, and incubated with shaking at 37°C at 140 rpm for 16 hours. Each strain was transferred to the same medium and cultivated another 6 hours. Then cells were harvested by centrifugation and suspended into LB medium (final OD₆₀₀=20) and mixed recipient cells, helper cells, and donor cells in 1:1:4 ratio (v/v/v).
The mixture was put on a nitrocellulose filter placed on LBMC agar plates. After these plates were incubated for 20 hours at 30 °C, cells on the filter were scratched and suspended in sterilized 0.85 % NaCl solution. The suspension was diluted appropriately and spread on LBMC plates containing 20 µg/ml of nalidixic acid (to select for *S*. *meliloti* IFO14782) and 10 µg/ml Tc (to select for pVK601). After incubation of these plates at 30°C for 5 days, colonies grown on the plates were picked up and cultured for plasmid extraction by QIAGEN plasmid mini kit. Obtained plasmid DNA was checked by treatment of restriction enzymes, subjected to agarose gel electrophoresis and showed an identical pattern to that of pVK601. The resulting colonies were purified by streaking on the same selection plate and used as recombinant strain *S*. *meliloti* IFO 14782/pVK601.
In a similar manner of construction of *S. meliloti* IFO 14782/pVK601 as mentioned above, *S. meliloti* IFO 14782/pVK602 and *S*. *meliloti* IFO 14782/pVK611 were obtained by using *E. coli* HB 101 carrying pVK602 and *E*. *coli* HB 101 carrying pVK611 as donor strains.
To confirm that expression of pdxJ and epd in recombinant strains, SDS-PAGE was performed. The cells were grown in LBMC medium with 10 µg/ml of Tc at 30°C for 18 hours. Then the grown cells were harvested by centrifugation, washed with saline and resuspended in the ice-cold 20 mM phosphate buffer (pH 8.2). The cell suspension was subjected to a sonicator (Bioruptor, Cosmo Bio Co. Japan) and the resultant lysate was centrifuged at 15,000 rpm for 10 min at 4°C to remove cell debris.
The supernatant was used as cell-free extract, and then subjected to SDS-PAGE in 12.5 % gel and stained by Comassie Brilliant Blue (Rapid Stain CBB Kit, nacalai tesque Japan). Expression of expected size of polypeptide (PdxJ 29.0 kDa, Epd 37.2 kDa) was detected in *S. meliloti* IFO 14782 having recombinant plasmids.

### Example 4: Production of vitamin B₆ by fermentation of S. meliloti IFO 14782 having recombinant plasmid

*S. meliloti* IFO14782 having a recombinant plasmid and the parent strain, *S. meliloti* IFO14782, were incubated on a LBMC agar plate at 30°C for 48 hours, and a loopful of each strain was inoculated to tubes containing 8 ml of a seed medium composed of 1% glucose, 1 % corn steep liquor (Nihon Syokuhin Kako Co., Ltd., Tokyo, Japan), 0.2 % Bacto yeast extract, 0.1 % Polypepton S (Nihon Pharmaceuticals Co. Japan), 0.05 % MgSO₄·7H₂O, 0.001 % MnSO₄·5H₂O, and 0.001 % FeSO₄·7H₂O, pH 6.8, and then the tubes were shaken on a reciprocal shaker (275 rpm) at 30°C.
After shaking for 19 hours, each 3 ml of culture broth was transferred to 500-ml flasks with two baffles containing 150 ml of a production medium composed of 6 % glucose, 3 % corn steep liquor, 0.8 % Bacto yeast extract, 0.175 % NH₄Cl, 0.05 % MgSO₄-7H₂O, 0.025 % MnSO₄·5H₂O, 1 % Allophosite (Shinagawa Chemicals Co., Ltd., Tokyo, Japan) and 0.025 % Actocol (Takeda Chemical Industries, Ltd., Osaka, Japan), pH 6.8, and shaken on a rotary shaker (180 rpm) at 30 °C. After shaking for 2 days, sterile solution of urea was added to the each flask at 0.125 %, and the shaking were further continued for 5 days. After cultivation for 7 days, contents of vitamin B₆ in the supernatant of each culture broth were quantified by HPLC and produced vitamin B₆ was calculated by the internal standard method with 4'-deoxypyridoxol as described below. To prepare the samples for HPLC, 400 µl of 500 mg/l of 4'-deoxypyridoxol as internal substance, 50 µl of 60 % perchloric acid, and 550 µl of deionized water was added to 250 µl of the standard solutions of pyridoxol or the supernatant from the culture broth, and then the mixture was put on the ice for 10 min. Then the mixture was centrifuged at 15,000 rpm and the supernatant was put on the following column.
The analytical conditions were as follows: column, Capcell pak C18 SG120 (4.6 x 250 mm) (Shiseido Co., Ltd., Tokyo, Japan); mobile phase, 0.1 M sodium perchlorate, 0.1 M potassium phosphate, and 2 % acetonitrile (pH 3.5); column temperature, 30 °C; flow rate, 1.0 ml/min; and detector, ultraviolet (at 292 nm). The results are shown in Table 1.
*S. meliloti* IFO14782/pVK601, the strain having the pdxJ expression plasmid, showed double higher titer than that of the parent strain IFO 14782, and *S. meliloti* IFO14782/pVK602, the strain having the epd expression plasmid, showed less titer than that of the parent strain. On the other hand, *S*. *meliloti* IFO14782/pVK611, the recombinant strain which incorporated both epd and pdxJ genes, produced 1,300 mg of pyridoxol per liter, and it was about 13 times higher titer than that of the parent strain. The productivity of vitamin B₆ was drastically improved by incorporating both epd and pdxJ genes.

**Table 1: PN productivity of recombinant S. meliloti**

| Strain | PN productivity (mg/L) |
|---|---|
| *S. meliloti* IFO14782 | 103 |
| *S. meliloti* IFO14782/pVK601 | 192 |
| *S. meliloti* IFO14782/pVK602 | 84 |
| *S. meliloti* IFO14782/pVK611 | 1,300 |

### Example 5: Isolation and purification of vitamin B₆ from culture broth

Vitamin B₆ was recovered from the culture broth of *S. meliloti* IFO14782/pVK611 prepared in the same cultural conditions as described in Example 4. The vitamin B₆ at each purification step and the concentration were followed by HPLC. One liter of the 168 hour-culture broth containing 1300 mg/L of vitamin B₆ was centrifuged at 7,500 rpm for 10 min. The pH of the resultant supernatant was adjusted to 3.1 with 1N hydrochloric acid, and then the supernatant was applied to a column (5.5 x 15 cm) packed with 350 ml of Amberlite CG 120 (H⁺ form, 100-200 mesh, Rohm and Haas Company, Philadelphia, Pennsylvania, USA). The column was washed with 500 ml of deionized water and then eluted with 5 % ammonium hydroxide. The vitamin B₆ fractions were concentrated under reduced pressure. The residue thus obtained was dissolved in 10 ml of deionized water, and the solution was charged on a column (5.5 x 16 cm) packed with 380 ml of Dowex 1 x 4 (OH- form, 200-400 mesh, Dow Chemical Co., Ltd., Midland, Michigan, USA), and then washed with 500 ml of deionized water. The column was then eluted with 0.1 N HCl. The fractions containing vitamin B₆ was concentrated to small volume under reduced pressure. After the solid residue was dissolved in small amount of hot ethanol, the solution was kept standing at 4 °C overnight. The resultant precipitates were collected by filtration and dried in vacuo to obtain 1,090 mg of crude crystals. It was recrystallized from ethanol to obtain 839 mg of white crystals having a melting point of 160 °C. The infrared absorption, ultra violet absorption, and NMR spectrum of the product coincided with those of authentic pyridoxol.

## Claims

1. A recombinant microorganism of the genus *Sinorhizobium* which comprises a vector containing pyridoxol 5'-phosphate synthase gene and D-erythrose 4-phosphate dehydrogenase gene, and being capable of producing vitamin B₆.

2. The microorganism according to claim 1, wherein the pyridoxol 5'-phosphate synthase gene is derived from *Escherichia coli* or *Sinorhizobium meliloti.*

3. The microorganism according to claim 1, wherein the pyridoxol 5'-phosphate synthase gene is derived from *Escherichia coli* K12 or *Sinorhizobium meliloti* IFO 14782.

4. The microorganism according to claim 1, wherein the pyridoxol 5'-phosphate synthase gene is derived from *Sinorhizobium meliloti* IFO 14782.

5. The microorganism according to claim 1, wherein the pyridoxol 5'-phosphate synthase gene is derived from *Escherichia coli* K12.

6. The microorganism according to claim 1, wherein the D-erythrose 4-phosphate dehydrogenase gene is derived from microorganism of *Escherichia coli* or *Vibrio cholerae.*

7. The microorganism according to claim 1, wherein the D-erythrose 4-phosphate dehydrogenase gene is derived from microorganism of *Escherichia coli* K12.

8. The microorganism according to claim 1 which is *Sinorhizobium meliloti* IFO 14782 comprising the plasmid pVK611 shown in figure 4.

9. A process for preparing vitamin B₆ by cultivating a recombinant microorganism of the genus *Sinorhizobium* which comprises a vector containing pyridoxol 5'-phosphate synthase gene and D-erythrose 4-phosphate dehydrogenase gene, and being capable of producing vitamin B₆ which comprises cultivating the recombinant microorganism under aerobic conditions at a pH value of about 5.0 to 9.0, at a temperature of 10°C to 40°C, and for 1 day to 15 days in a medium containing an assimilable carbon source, a digestible nitrogen source, inorganic salts, and other nutrients necessary for the growth of the microorganism, and then recovering vitamin B₆ formed and accumulated in the culture broth.

10. The process according to claim 9, wherein the pyridoxol 5'-phosphate synthase gene is derived from *Escherichia coli* or *Sinorhizobium meliloti.*

11. The process according to claim 9, wherein the pyridoxol 5'-phosphate synthase gene is derived from *Escherichia coli* K12 or *Sinorhizobium meliloti* IFO 14782.

12. The process according to claim 9, wherein the pyridoxol 5'-phosphate synthase gene is derived from *Sinorhizobium meliloti* IFO 14782.

13. The process according to claim 9, wherein the pyridoxol 5'-phosphate synthase gene is derived from *Escherichia coli* K12.

14. The process according to claim 9, wherein the D-erythrose 4-phosphate dehydrogenase gene is derived from microorganism of *Escherichia coli* or *Vibrio cholerae.*

15. The process according to claim 9, wherein the D-erythrose 4-phosphate dehydrogenase gene is derived from microorganism of *Escherichia coli* K12.

16. The process according to claim 9, wherein the recombinant microorganism is *Sinorhizobium meliloti* IFO 14782/pVK611.

## Patentansprüche

1. Rekombinanter Mikroorganismus der Gattung *Sinorhizobium,* der einen Vektor enthält, der Pyridoxol-5'-phosphat-Synthasegen und D-Erythrose-4-phosphat-Dehydrogenasegen enthält und Vitamin B₆ erzeugen kann.

2. Mikroorganismus nach Anspruch 1, wobei das Pyridoxol-5'-phosphat-Synthasegen aus *Escherichia coli* oder *Sinorhizobium meliloti* stammt.

3. Mikroorganismus nach Anspruch 1, wobei das Pyridoxol-5'-phosphat-Synthasegen aus *Escherichia coli* K12 oder *Sinorhizobium meliloti* IFO 14782 stammt.

4. Mikroorganismus nach Anspruch 1, wobei das Pyridoxol-5'-phosphat-Synthasegen aus *Sinorhizobium meliloti* IFO 14782 stammt.

5. Mikroorganismus nach Anspruch 1, wobei das Pyridoxol-5'-phosphat-Synthasegen aus *Escherichia coli* K12 stammt.

6. Mikroorganismus nach Anspruch 1, wobei das D-Erythrose-4-phosphat-Dehydrogenasegen aus einem Mikroorganismus von *Escherichia coli* oder *Vibrio cholerae* stammt.

7. Mikroorganismus nach Anspruch 1, wobei das D-Erythrose-4-phosphat-Dehydrogenasegen aus einem Mikroorganismus von *Escherichia coli* K12 stammt.

8. Mikroorganismus nach Anspruch 1, der *Sinorhizobium meliloti* IFO 14782 ist, der das Plasmid pVK611, das in Fig. 4 gezeigt ist, aufweist.

9. Verfahren zur Herstellung von Vitamin B₆ durch Züchtung eines rekombinanten Mikroorganismus der Gattung *Sinorhizobium,* der einen Vektor enthält, der Pyridoxol-5'-phosphat-Synthasegen und D-Erythrose-4-phosphat-Dehydrogenasegen enthält und Vitamin B₆ erzeugen kann, das umfasst, dass der rekombinante Mikroorganismus unter aeroben Bedingungen bei einem pH-Wert von etwa 5,0 bis 9,0 bei einer Temperatur von 10 bis 40°C 1 bis 15 Tage lang in einem Medium gezüchtet wird, das eine assimilierbare Kohlenstoffquelle, eine verdaubare Stickstoffquelle, anorganische Salze und andere Nährstoffe, die fiir das Wachstum des Mikroorganismus notwendig sind, enthält, und dann das gebildete und in der Kulturbrühe angesammelte Vitamin B₆ gewonnen wird.

10. Verfahren nach Anspruch 9, wobei das Pyridoxol-5'-phosphat-Synthasegen aus *Escherichia coli* oder *Sinorhizobium meliloti* stammt.

11. Verfahren nach Anspruch 9, wobei das Pyridoxol-5'-phosphat-Synthasegen aus *Escherichia coli* K12 oder *Sinorhizobium meliloti* IFO 14782 stammt.

12. Verfahren nach Anspruch 9, wobei das Pyridoxol-5'-phosphat-Synthasegen aus *Sinorhizobium meliloti* IFO 14782 stammt.

13. Verfahren nach Anspruch 9, wobei das Pyridoxol-5'-phosphat-Synthasegen aus *Escherichia coli* K12 stammt.

14. Verfahren nach Anspruch 9, wobei das D-Erythrose-4-phosphat-Dehydrogenasegen aus einem Mikroorganismus von *Escherichia coli* oder *Vibrio cholerae* stammt.

15. Verfahren nach Anspruch 9, wobei das D-Erythrose-4-phosphat-Dehydrogenasegen aus einem Mikroorganismus von *Escherichia coli* K12 stammt.

16. Verfahren nach Anspruch 9, wobei der rekombinante Mikroorganismus *Sinorhizobium meliloti* IFO 14782/pVK611 ist.

## Revendications

1. Microorganisme recombiné du genre *Sinorhizobium* qui comprend un vecteur contenant le gène de la pyridoxol 5'-phosphate synthase et le gène de la D-érythrose 4-phosphate déshydrogénase et qui est capable de produire de la vitamine B₆.

2. Microorganisme selon la revendication 1, dans lequel le gène de la pyridoxol 5'-phosphate synthase dérive *d'Escherichia coli* ou de *Sinorhizobium meliloti.*

3. Microorganisme selon la revendication 1, dans lequel le gène de la pyridoxol 5'-phosphate synthase dérive *d'Escherichia coli* K12 ou de *Sinorhizobium meliloti* IFO 14782.

4. Microorganisme selon la revendication 1, dans lequel le gène de la pyridoxol 5'-phosphate synthase dérive de *Sinorhizobium meliloti* IFO 14782.

5. Microorganisme selon la revendication 1, dans lequel le gène de la pyridoxol 5'-phosphate synthase dérive *d'Escherichia coli* K12.

6. Microorganisme selon la revendication 1, dans lequel le gène de la D-érythrose 4-phosphate déshydrogénase dérive du microorganisme *Escherichia coli* ou *Vibrio cholerae.*

7. Microorganisme selon la revendication 1, dans lequel le gène de la D-érythrose 4-phosphate déshydrogénase dérive du microorganisme *Escherichia coli* K12.

8. Microorganisme selon la revendication 1 qui est *Sinorhizobium meliloti* IFO 14782 comprenant le plasmide pVK611 représenté sur la figure 4.

9. Procédé pour préparer de la vitamine B₆ en cultivant un microorganisme recombiné du genre *Sinorhizobium* qui comprend un vecteur contenant le gène de la pyridoxol 5'-phosphate synthase et le gène de la D-érythrose 4-phosphate déshydrogénase et qui est capable de produire de la vitamine B₆, qui comprend la culture du microorganisme recombiné dans des conditions aérobies à une valeur de pH d'environ 5,0 à 9,0, à une température de 10 °C à 40 °C et pendant 1 à 15 jours dans un milieu contenant une source de carbone assimilable, une source d'azote digestible, des sels inorganiques et d'autres nutriments nécessaires à la croissance du microorganisme et qui comprend ensuite la récupération de la vitamine B₆ qui s'est formée et accumulée dans le bouillon de culture.

10. Procédé selon la revendication 9, dans lequel le gène de la pyridoxol 5'-phosphate synthase dérive *d'Escherichia coli* ou de *Sinorhizobium meliloti.*

11. Procédé selon la revendication 9, dans lequel le gène de la pyridoxol 5'-phosphate synthase dérive *d'Escherichia coli* K12 ou de *Sinorhizobium meliloti* IFO 14782.

12. Procédé selon la revendication 9, dans lequel le gène de la pyridoxol 5'-phosphate synthase dérive de *Sinorhizobium meliloti* IFO 14782.

13. Procédé selon la revendication 9, dans lequel le gène de la pyridoxol 5'-phosphate synthase dérive *d'Escherichia coli* K12.

14. Procédé selon la revendication 9, dans lequel le gène de la D-érythrose 4-phosphate déshydrogénase dérive du microorganisme *Escherichia coli* ou *Vibrio cholerae.*

15. Procédé selon la revendication 9, dans lequel le gène de la D-érythrose 4-phosphate déshydrogénase dérive du microorganisme *Escherichia coli* K12.

16. Procédé selon la revendication 9, dans lequel le microorganisme recombiné est *Sinorhizobium meliloti* IFO 14782/pVK611.
